(19) Europäisches Patentamt
European Patent Office
Office européen des brevets

(11) Veröffentlichungsnummer: **0 077 503**
**A1**

# (12) EUROPÄISCHE PATENTANMELDUNG

(21) Anmeldenummer: **82109320.0**

(22) Anmeldetag: **08.10.82**

(51) Int. Cl.[3]: **C 07 D 317/12**
C 11 B 9/00

(30) Priorität: **17.10.81 DE 3141327**

(43) Veröffentlichungstag der Anmeldung:
**27.04.83 Patentblatt 83/17**

(84) Benannte Vertragsstaaten:
**AT BE CH DE FR GB IT LI LU NL**

(71) Anmelder: **Henkel Kommanditgesellschaft auf Aktien**
**Postfach 1100 Henkelstrasse 67**
**D-4000 Düsseldorf 1(DE)**

(72) Erfinder: **Bruns, Klaus, Dr.**
**Notburgaweg 6**
**D-4150 Krefeld-Traar(DE)**

(72) Erfinder: **Meffert, Alfred, Dr.**
**Marie-Curie-Strasse 10**
**D-4019 Monheim(DE)**

(54) **2-(1-Isopropyl-4-methyl-bicyclo(3.1.0)hexyl-3-yl)-1,3-dioxolane, deren Herstellung und Verwendung als Riechstoffe, sowie diese enthaltende Riechstoffkompositionen.**

(57) Beansprucht werden 2-(1-Isopropyl-4-methyl-bicyclo [3.1.0]hexyl-3-yl)-1,3-dioxolane, deren Herstellung und Verwendung als Riechstoffe oder in Riechstoffkompositionen.

EP 0 077 503 A1

Croydon Printing Company Ltd

0077503

HENKEL KGaA
ZR-FE/Patente
Dr. Bz/Br

4000 Düsseldorf, den 14.10.1981

P a t e n t a n m e l d u n g

D 6397 EP

"2-(1-Isopropyl-4-methyl-bicyclo [3.1.0] hexyl-3-yl)-1,3-dioxolane, deren Herstellung und Verwendung als Riechstoffe, sowie diese enthaltende Riechstoffkompositionen"

Es wurde gefunden, daß 2-(1-Isopropyl-4-methyl-bicyclo [3.1.0] hexyl-3-yl)-1,3-dioxolane wertvolle neue Riechstoffe darstellen, die insbesondere für neuartige Riechstoffkompositionen mit besonderer Duftnote geeignet sind. Die Verbindungen besitzen die nachstehenden Formeln:

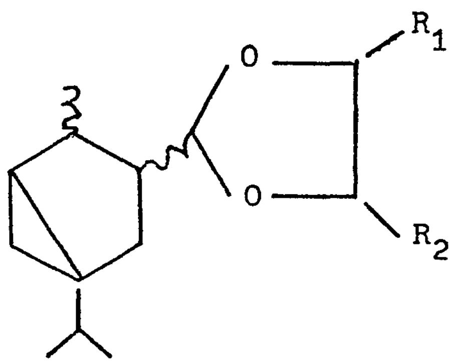

(1) $R_1 = R_2 = H$
(2) $R_1 = H$; $R_2 = CH_3$
(3) $R_1 = R_2 = CH_3$

Die Herstellung der Verbindungen gelingt nach an sich bekannten Methoden durch Hydroformylierung von Thujen (4) mit $H_2$/CO unter Druck zum Thujen-3-carbaldehyd (5) und nachfolgender Umsetzung mit Ethandiol, Propandiol-1,2 oder Butandiol-2,3 zu den entsprechenden Dioxolanen (1-3):

...

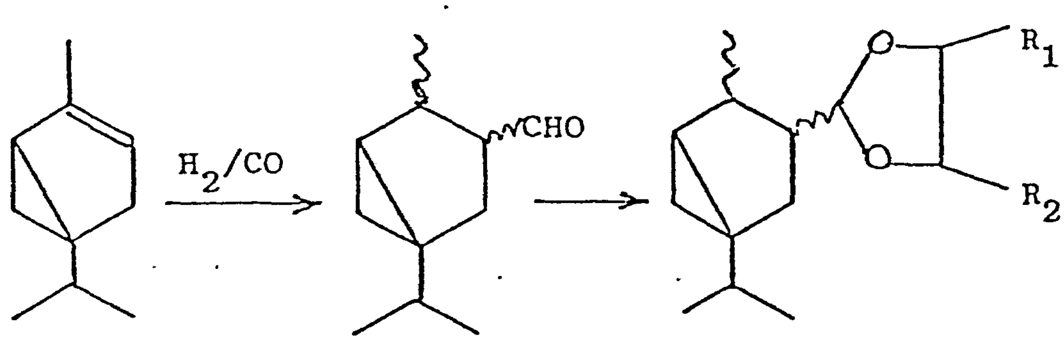


(4) (5) (1-3)

Die erhaltenen Rohprodukte werden zweckmäßig durch Destillation gereinigt.

Das Dioxolan (1) weist einen animalisch, holzigen Geruch mit Castoreum-Note auf, das Dioxolan (2) ist im Geruch holzig mit Isononylalkohol-Note, das Dioxolan (3) besitzt einen blumigen Geruch mit Firnis- beziehungsweise Lack-Note.

Die neuen Riechstoffe zeichnen sich durch eine sehr gute Kombinationsfähigkeit mit anderen, natürlichen und synthetischen Riechstoffen zu neuen, interessanten Duftkompositionen aus. Sie lassen sich mit anderen Riechstoffen in verschiedenen Mengenverhältnissen mischen. Im allgemeinen liegt ihr Anteil in Riechstoffkompositionen im Bereich von 1 bis 50 Gewichtsprozent, bezogen auf die gesamte Komposition. Solche Riechstoffkompositionen können zur Parfümierung von kosmetischen Präparaten wie Duftwässern, Haarwässern, Aerosolpräparaten, Desodorantien, Cremes, Lotionen, Badepräparaten, Shampoos, Toilettenseifen sowie auch in der Extrait-Parfümerie verwendet werden. Sie können aber auch zur Geruchsverbesserung technischer Produkte, zum Beispiel von Wasch- und Reinigungsmitteln, Wäscheweichspülmitteln, Textil-

...

behandlungsmitteln und so weiter eingesetzt werden. Zur Parfümierung der verschiedenen Produkte werden diese Kompositionen im allgemeinen in Konzentrationen von 0,05 bis 2,0 Gewichtsprozent, bezogen auf das gesamte Produkt, zugesetzt.

...

## Beispiele

1. Herstellung von Thujan-3-carbaldehyd (5)

In einen 1 l-Rührautoklav wurden 300 g Thujen (4) (91,2 % Thujen/8,3 % α-Pinen (GC); $\alpha_D^{21}$ + 16,4°), 12 g Triphenylphosphin und 0,6 g Rhodiumcarbonylchlorid-ditriphenylphosphin-Katalysator eingebracht und jeweils 100 bar Wasserstoff bzw. Kohlenmonoxid aufgedrückt.

Anschließend wurde innerhalb einer Stunde auf 170 °C erhitzt und 6 1/2 bis 7 Stunden bei dieser Temperatur gerührt. Die Heizung wurde abgestellt, als der Druck konstant blieb.

Nach Abkühlen wurde der Autoklav mit Stickstoff gespült. Das Rohprodukt wurde filtriert (Rohausbeute 360 g) und anschließend im Wasserstrahlvakuum destilliert.

Es wurden 268 g Thujanaldehyd (5) entsprechend 73 % der Theorie mit folgenden physikalischen Daten erhalten:

$Kp_{14-15}$ 80-103°, GC: 75-86 % Thujan-3-carbaldehyd (5)

$n_D^{20}$ : 1,4572; $\alpha_D^{25}$ : - 37°

IR (Film): 2720, 1725/cm (CHO); 1360, 1380/cm (i-Propyl)

3050, 1020/cm (Cyclo-$C_3$)+

MS : m/e 166 (Hauptkomponente)

+nach V.K. BALAKRISHNAN et. al, Perfumery and Essential Oil Rec. 47, 383 (1956)

...

2. 2-(1-Isopropyl-4-methyl-bicyclo[3.1.0]hexyl-3-yl)-1,3-dioxolan (1)

Zu einer Mischung von 5,6 g des nach Beispiel 1 hergestellten Thujan-3-carbaldehyds (5), 4,99 g Orthoameisensäure-triethylester und 2,09 g Ethandiol (Molverhältnis 1:1:1) wurden unter Rühren 50 mg p-Toluolsulfonsäure zugesetzt, worauf leichte Erwärmung eintrat. Nach Rühren über Nacht bei Raumtemperatur wurden Ameisensäure-ethylester und Ethanol abdestilliert. Der in Ether aufgenommene Rückstand wurde mit 2n-Sodalösung, anschließend mit Wasser neutral gewaschen und über wasserfreiem Natriumsulfat getrocknet. Nach Abdestillieren des Lösungsmittels hinterblieben 5,7 g (80,5 % der Theorie) Rohprodukt, das nach Destillation ein Produkt mit folgenden Daten liefert:

$Kp_{2,5}$ 99-100 °C; GC: 91 %; $\alpha_D^{25}$ - 32,8°; $n_D^{20}$ 1,4660

IR (Film): 1160, 1130, 1055/cm (O-C-O); 1380/1360/cm (i-Propyl), 3058, 1020/cm (Cyclo-$C_3$)

$^1$H-NMR($CDCl_3$): 4,72 ppm (d,1H)(O-C$\underline{H}$-O); 3,85 ppm (m,4H) (O-C$\underline{H}_2$-C$\underline{H}_2$-O); 2,10 ppm (m,1H)($C_3$-H); 1,05/0,97 ppm (d,3H) ($C_4$-C$\underline{H}_3$); 0,94/0,86/0,79 ppm (m,6H) ($C_1$-CH(C$\underline{H}_3$)$_2$); ca. 0,12 ppm (m,3H) ($C_5$-H/$C_6$-H)

| $C_{13}H_{22}O_2$ (210,32) | | Ber. | Gef. |
|---|---|---|---|
| | MG | 210 | 210 (MS) |
| | C | 74,21 | 74,0 |
| | H | 10,54 | 10,4 |
| | O | 15,21 | 15,2 |

Geruch: animalisch, holzig, Castoreum-Note ...

3. 2-(1-Isopropyl-4-methyl-bicyclo [3.1.0] hexyl-3-yl)-1.3-dioxolan-4(5)-methyl (2)

Die Herstellung erfolgt durch Umsetzung von Thujan-3-carbaldehyd (5) mit Propandiol-1,2 wie im Beispiel 2 angegeben.

$Kp_{1,5}$ 91-93 °C; GC: 90 % (Isomerengemisch)
$n_D^{20}$ 1,4598; $\alpha_D^{25}$ - 29,8°

IR (Film): 1160,1127,1050/cm (O-C-O); 1376,1358/cm (i-Propyl + sek.-Methyl)
3057,1020/cm (Cyclo-$C_3$)

GC/MS: 4 Isomere, m/e jeweils 224

Geruch: holzig, Isononylalkohol-Note

4. 2-(1-Isopropyl-4-methyl-bicyclo [3.1.0] hexyl-3-yl)-1.3-dioxolan-4,5-dimethyl (3)

Die Herstellung erfolgt durch Umsetzung von Thujan-3-carbaldehyd (5) mit Butandiol-2,3 wie im Beispiel 2 angegeben.

$Kp_{2,5}$ 97-99 °C; GC: 94 % (Isomerengemisch)
$n_D^{20}$ 1,4582; $\alpha_D^{25}$ - 28,0°

IR (Film): 1060-1155/cm (O-C-O); 1360,1375/cm (i-Propyl + sek.-Methyl)
3056,1020/cm (Cyclo-$C_3$)

GC/MS: 4 Isomere, m/e jeweils 238

Geruch: blumig, Firnis-, Lack-Note

...

5. <u>Nostalgische Komposition für Extraitparfüm bzw. Eau de Toilette</u>

| | | | |
|---|---|---|---|
| 2-(1-Isopropyl-4-methyl-bicyclo [3.1.0] hexyl-3-yl)-1,3-dioxolan (1) | | 100 Gew.-Teile | |
| 13-Oxabicyclo [10.3.0] Pentadecan (Cyclamber ®) | | 100 " | " |
| α-iso-Methyljonon | | 80 " | " |
| Bergamottöl | | 60 " | " |
| Hydroxycitronellal | | 50 " | " |
| Benzylsalicylat | | 50 " | " |
| α-Hexylzimtaldehyd | | 40 " | " |
| Ylang-Ylang | | 40 " | " |
| Isoeugenol | | 40 " | " |
| Moschus Ambrette | | 40 " | " |
| Moschus Keton | | 40 " | " |
| Vetiverylacetat | | 40 " | " |
| Sandelholzöl, ostindisch | | 40 " | " |
| Phenylethanol | | 40 " | " |
| Aldehyd C 14 sogen. | 10 % in DEP | 30 " | " |
| Eugenol | | 30 " | " |
| Styrax Resin | | 30 " | " |
| Benzol Siam Resin | | 30 " | " |
| δ-Decalacton | 10 % in DEP | 20 " | " |
| Jasmin Absolue | 10 % in DEP | 20 " | " |
| Geraniol | | 20 " | " |
| Amylsalicylat | | 20 " | " |
| Patchouliöl | | 10 " | " |
| Cyclopentadecanolid | | 10 " | " |
| Undecylenaldehyd | 10 % in DEP | 10 " | " |
| Eichenmoos Absolue | | 5 " | " |
| Zimtöl Ceylon | | 5 " | " |
| | | 1000 Gew.-Teile | |

...

## P a t e n t a n s p r ü c h e

1. 2-(1-Isopropyl-4-methyl-bicyclo [3.1.0] hexyl-3-yl)-1,3-dioxolane der Formel

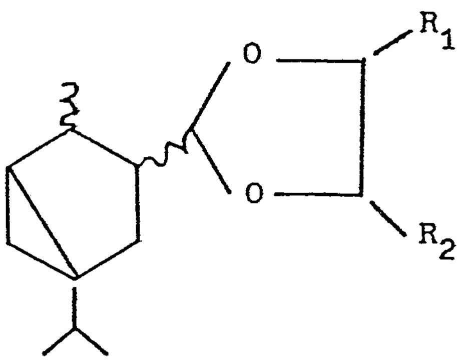

(1) $R_1 = R_2 = H$
(2) $R_1 = H$; $R_2 = CH_3$
(3) $R_1 = R_2 = CH_3$

2. 2-(1-Isopropyl-4-methyl-bicyclo [3.1.0] hexyl-3-yl-1,3-dioxolane der Formeln (1), (2) und (3) gemäß Anspruch 1 in Form der Isomerengemische.

3. Herstellung der 2-(1-Isopropyl-4-methyl-bicyclo [3.1.0] hexyl-3-yl)-1,3-dioxolane des Anspruchs 1 und 2 durch Hydroformylierung von Thujen mit $H_2$/CO unter Druck zum Thujan-3-carbaldehyd und nachfolgende Umsetzung mit Ethandiol, Propandiol-1,2 oder Butandiol-2,3 zu den Dioxolanen (1-3).

4. Verwendung der 2-(1-Isopropyl-4-methyl-bicyclo [3.1.0] hexyl-3-yl)-1,3-dioxolane nach Anspruch 1 und 2 als Riechstoffe.

5. Riechstoffkompositionen, dadurch gekennzeichnet, daß sie 2-(1-Isopropyl-4-methyl-bicyclo [3.1.0] hexyl-3-yl)-1,3-dioxolane des Anspruchs 1 und 2 in einer Menge von 1-50 Gewichtsprozent, bezogen auf die gesamte Komposition, enthalten.

Europäisches Patentamt

**EUROPÄISCHER RECHERCHENBERICHT**

0077503
Nummer der Anmeldung

EP 82 10 9320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CHEMICAL ABSTRACTS, Band 96, 30. Juni 1982, Seite 608, Nr. 85751a, Columbus, Ohio, USA M. WALKOWICZ et al.: "Synthesis and odor characteristics of some 6,6-dimethylbicyclo[3.1.0] hexane derivatives" & PERFUM. FLAVOR. 1981, 6(4), 21-2, 24 & CHEMICAL SUBSTANCE INDEX, Ch-Io, 30. June 1982, Nr. 2384CS, The American Chemical Society * Zusammenfassungen * | 1,4,5 | C 07 D 317/12<br>C 11 B 9/00 |
| A | US-A-3 748 344 (D. McCLOUD, A. SCHLEPPNIK) * Spalten 1-6 * | 1,3-5 | |
| A | EP-A-0 033 928 (HENKEL) * Ansprüche * | 1,4,5 | RECHERCHIERTE SACHGEBIETE (Int. Cl. 3)<br>C 07 D 317/00 |

Der vorliegende Recherchenbericht wurde für alle Patentansprüche erstellt.

| Recherchenort | Abschlußdatum der Recherche | Prüfer |
|---|---|---|
| DEN HAAG | 21-01-1983 | FRANCOIS J.C.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82